# EUROPEAN PATENT APPLICATION

(11) **EP 4 361 132 A1**
(43) Date of publication of application: **01.05.2024**
(21) Application number: 22830362.4
(22) Date of filing: 23.06.2022
(51) Int. Cl.: C07D 295/24, D01F 11/00, D01F 13/00

(54) **PURIFICATION METHOD AND SYSTEM FOR N-METHYLMORPHOLINE-N-OXIDE, AND OBTAINED N-METHYLMORPHOLINE-N-OXIDE**

(30) Priority: 02.07.2021 CN 202110748233
(71) Applicant: B-FCTL Co., Ltd., Cangzhou, Hebei 061100 (CN)
(72) Inventor: LU, Wanli, Hebei 061100 (CN); MA, Jie, Shaanxi 710018 (CN)
(74) Representative: Müller Schupfner & Partner Patent- und Rechtsanwaltspartnerschaft mbB
(86) International application number: PCT/CN2022/100880
(87) International publication number: WO 2023/274038

(57) **Abstract**

The invention discloses a purification method and system of N-methylmorpholine N-oxide (NMMO), and a N-methylmorpholine N-oxide obtained thereof. The invention is used for recovering and purifying NMMO in a lyocell fiber coagulation bath. The method comprises: performing flocculation, microfiltration, ultrafiltration and nanofiltration membrane separation process to the lyocell fiber coagulation bath to remove macromolecular impurities such as suspending substance, heavy metal ions, heavy metal complexes, polysaccharides, etc., and then performing cooling crystallization between -20°C and 78°C to obtain NMMO hydrate crystals. The NMMO hydrate crystals obtained by the method provided in the invention do not contain PG, PG oxidation products, cellulose breakdown products such as various carbohydrates, sugar ketones, sugar acid, furans, furfurals, phenols, and NMMO decomposition products, which are present in the coagulation bath. The NMMO hydrate crystals do not contain various metal ions and anions as well. The NMMO hydrate crystals are added with water to prepare a NMMO aqueous solution with a concentration of 19.8%, and the electrical conductivity is about 14.8µs/cm. The crystalline NMMO obtained by a first crystallization is added with water to perform crystallization again, and a crystalline NMMO with higher purity can be obtained. The crystalline NMMO with higher purity is added with water to prepare a NMMO aqueous solution with a concentration of 19.8% and the electrical conductivity is reduced to less than 12.4µs/cm.

## Description

### FIELD

The invention relates to a purification method of N-methylmorpholine N-oxide, a purification system of N-methylmorpholine N-oxide and a N-methylmorpholine N-oxide obtained using the method and the system.

### BACKGROUND

Lyocell fibers is a synthetic cellulose fiber obtained by dissolving cellulose pulp directly in a mixed solvent of methylmorpholine oxide (N-methylmorpholine N-oxide, NMMO) and water and performing dry-jet wet spinning. The dilute NMMO aqueous solution obtained after the precipitation of the lyocell fibers is known as lyocell fiber coagulation bath or discharge liquid of the lyocell fiber coagulation bath. The lyocell fibers have excellent properties, its raw materials are renewable, the production process is simple, the NMMO solvent used is non-toxic, the product waste is biodegradable and does not pollute the environment. Therefore, the lyocell fibers are known as green and environmentally friendly fibers with good development prospect in the 21^{st} century.

At present, lyocell fiber companies basically use coniferous forest pulp containing 3-5% of hemicellulose to produce the lyocell fiber, and functional fibers can be spun by additionally adding some other special substances or replacing coniferous forest pulp with other special pulp in NMMO spinning solution that dissolves cellulose fibers. US patent No.9,441,318 uses bamboo pulp to prepare odor-resistant lyocell fibers; US patent No.9,845,575 indicates that by dissolving hemicellulose in the lyocell spinning solution, the spun lyocell fibers can alleviate the fibrillation problem of lyocell fibers to a large extent. Another patent (US8,092,732) mentions that by adding chitosan to the lyocell spinning solution, the spun lyocell fibers have antibacterial and deodorizing properties; if graphene (US 10,351,971), kaolin (US 10,400,356), pearl powder (US 8,633,120) and other inorganic functional powder materials are added into the lyocell spinning solution, functional lyocell fibers with excellent thermal conductivity, flame retardancy, and beautiful glitter can be spun respectively.

The production process of the above-mentioned lyocell fiber is divided into two parts. One part of the production process is cellulose dissolution and spinning in NMMO and the other part is purification and recovery of the NMMO solvent in the lyocell fiber coagulation bath or discharge liquid of the lyocell fibers coagulation bath after lyocell spinning.

In addition, NMMO solution that dissolves cellulose can be used to prepare lyocell thin films and photographic films (US7,938,993), lyocell non-woven fabrics (US8,420,004), material for cigarette filter (US10,306,919), etc. These production processes also need to purify and recover the NMMO solvent in the lyocell coagulation bath after the corresponding lyocell products are manufactured.

Specifically, in lyocell production, the cellulose pulp is first dissolved in a NMMO aqueous solution with a mass concentration above 85% at 100-110°C to prepare a lyocell spinning solution with cellulose content of 10-14%, then the corresponding cellulose products are produced by dry-jet, wet spinning or molding in water and in the meantime, the resulting lyocell fiber coagulation bath has 10-25%(w/w) concentration of NMMO. During the dissolution of the pulp in the NMMO, the NMMO and the cellulose will have complex chemical decomposition reactions in the heating state and produce multiple impurities. To recycle the NMMO, various impurities dissolved in the lyocell coagulation bath need to be removed. Taking the spinning of lyocell staple fiber, which is currently the most commonly used, as an example, the NMMO mass concentration in the coagulation bath after lyocell spinning is between 10-25%, the color is brownish yellow or even soy sauce color and the pH value is 7.5-9. The dissolution of the pulp in the NMMO needs to be carried out at higher temperatures while the NMMO may decompose into impurities such as N-methylmorpholine, morpholine, formaldehyde, chemical compounds with large π electron-conjugated structure, etc. at high temperature. Highly reactive free radicals may also be produced during the decomposition, which leads to oxidation of the cellulose and breakdown and decomposition of cellulose chain. Metal ions such as copper and iron ions in the cellulose solution can catalyze and accelerate the decomposition of NMMO and the breakdown of the cellulose and deepen the color of the cellulose solution. Cellulose breakdown products include oligosaccharide with more than two saccharides and monosaccharide, and carbohydrates may decompose into a dozen of impurities such as saccharon, saccharic acid, furans, furfurals, phenols, etc. in the meantime. There may be further reactions between these breakdown products and it has been disclosed that up to 20 organic impurities containing chromophores may be produced (Rosenua T., Potthast A., Milacher W., Adorjan I., Hofinger A. and Kosma P., Discoloration of cellulose solutions in N-methylmorpholine-N-oxide (Lyocell). Part 2: Isolation and identification of chromophores. Cellulose 2005,12:197-208) (ZHENG Yu-cheng, Cause analysis of color change in lyocell fiber production, Synthetic Fiber, 2018, 47 (8): 17-23).

If the above impurities cannot be removed during the recycling of the NMMO in the coagulation bath, the accumulation of the impurities will have a negative impact on spinning and especially cause greater damage to the production of lyocell filament; To prevent NMMO decomposition and cellulose breakdown, multiple compound stabilizers including propyl gallate (PG) and hydroxylamine need to be added during the dissolution of cellulose by NMMO. These stabilizers, as well as the oxidized products of propyl gallate (PG) from scavenging free radicals and the condensation products from the reaction of PG with formaldehyde all have darker colors. Excessive residues of these chromophores in the solution will deepen the color of the lyocell fiber and as a result, will affect the whiteness of the lyocell fiber product. In addition, to remove suspending residual substance in the NMMO of the lyocell coagulation bath and to reduce solution turbidity, polyacrylamide (PAM) flocculant is usually added in the lyocell production to perform flocculation and sedimentation and in a result, the suspending residual substance is then removed by the following filtration. However, there is still a certain amount of hemicellulose, oligosaccharides, etc. which have not been removed in the lyocell coagulation bath. Also, there is still a small amount of PAM residue in the NMMO solution of the lyocell coagulation bath. Therefore, the impurities in the lyocell coagulation bath are complex, and the above impurities in the NMMO solvents of the coagulation bath must be purified and removed when the NMMO solvents are recovered for recycling.

The current purification and recovery of the NMMO in the coagulation bath mainly use anion-exchange resin and cation-exchange resin but the purification and recovery by using ion-exchange resin have many drawbacks: the recovered and purified NMMO still has many impurities and the purity of the recovered NMMO is relatively low. A number of impurities in the coagulation bath cannot form ions in acid-base environment, such as propyl gallate (PG) and products of its reaction, carbohydrate and decomposition products, and thermal decomposition products of the NMMO, therefore the impurities cannot be completely removed by ion exchange resin. A certain amount of impurities will still remain in the NMMO purified and recovered only by ion-exchange resin, and the NMMO purified and recovered by this method is likely to affect the lyocell filament production. The applicant of the invention has performed the comparative HPLC analysis of the lyocell coagulation baths before and after the purification using ion-exchange resin from many Chinese lyocell manufacturers and it has been discovered that the coagulation baths contain multiple impurities including PG oxides which cannot be removed by ion-exchange resin. Besides, many other impurities such as PG cannot be completely removed by ion-exchange resin and the purity of NMMO after purification by ion-exchange resin alone is not high accordingly.

Moreover, most lyocell manufacturers are currently only able to use coniferous forest pulps with a very low hemicellulose content, while cheap and easily obtained broad-leaved forest pulps are not able to be used since the broad-leaved forest pulps contain higher hemicellulose content. The hemicellulose decomposes more easily in the lyocell production to generate more impurities which are largely accumulated in the purified and recovered NMMO and seriously affect the stable production of lyocell as a result. If easily decomposable substances or inorganic functional materials, such as chitosan, hemicellulose, kaolin, graphene, pearl powder, are added in the lyocell spinning solution, more impurities will be produced during the spinning process due to these added materials. The method of purification and recovery of NMMO by using ion-exchange resin alone is not able to remove these impurities, and in a result, these impurities will gradually accumulate in the spinning system, and when these impurities accumulate to a certain amount, the production of the above lyocell functional fibers may seriously be affected.
(2) The amount of wastewater with high concentration of salt and COD content produced in the regeneration of anion-exchange resin and cation-exchange resin is significant and there exists severe environmental problems. Exhausted ion-exchange resins need to be regenerated, which means cation exchange resins require a 4-6% HCl aqueous solution that is 4-6 times the volume of the regenerated resins, while anion exchange resins require a 4-6% NaOH aqueous solution that is 4-6 times the volume of the regenerated resins. The wastewater from the resin regeneration contains not only high content of salt but also a significant amount of organic impurities, which leads to high treatment costs. Generally, producing 1 ton of lyocell fibers will have to generate 10-15 tons of the resin regeneration wastewater. Comparing with viscose fibers, although the amount of wastewater generated in the lyocell fiber production is only 10% of the wastewater generated in the viscose fiber production, the amount of wastewater generated is still very significant.
(3) Relatively high production costs. Ion-exchange resins suitable for purification of NMMO in the lyocell coagulation bath are expensive and have lifespan of only 1-2 years. The cost of lyocell fibers apportioned per ton is 500 CNY. According to the national hazardous waste classification list, the spent ion-exchange resins are classified as hazardous waste and must be disposed of at high costs by qualified environmental protection companies. Treatment cost of wastewater with high concentration of salt and COD content generated by regeneration of the exhausted ion-exchange resins is also very expensive.

Hence, in the face of increasingly severe environmental protection situation and production cost pressure, lyocell production urgently requires a cleaner, more environmentally friendly, more economical, more efficient purification and recovery method of NMMO in the coagulation bath.

### SUMMARY

A main object of the invention is to provide a purification method and system of N-methylmorpholine N-oxide (NMMO) and a NMMO obtained thereof to overcome defects of less economical and less environmentally friendly purification method of NMMO in the lyocell fiber coagulation bath in the prior art, as well as defect of low purity of the NMMO obtained by purification.

To achieve the above objects, the invention provides a purification method of NMMO, which is used to purify NMMO in a lyocell fiber coagulation bath, comprising the steps of: performing cooling crystallization to the lyocell fiber coagulation bath between -20 °C and 78 °C to obtain NMMO hydrate crystals.

The purification method of NMMO in the invention, wherein comprising another step before performing the cooling crystallization to the lyocell fiber coagulation bath: removing carbohydrates in the lyocell fiber coagulation bath.

The purification method of NMMO in the invention, wherein comprising another step before performing the cooling crystallization to the lyocell fiber coagulation bath: performing at least one of the microfiltration, ultrafiltration and nanofiltration to the lyocell fiber coagulation bath.

The purification method of NMMO in the invention, wherein comprising another step before performing the cooling crystallization to the lyocell fiber coagulation bath: mixing the lyocell fiber coagulation bath with coagulant aids to obtain a mixture to perform flocculation and sedimentation; then performing at least one of the microfiltration, ultrafiltration and nanofiltration to the mixture.

The purification method of NMMO in the invention, wherein concentrating the lyocell fiber coagulation bath before the cooling crystallization is performed until the mass concentration of NMMO in the lyocell fiber coagulation bath is 56.5% ~ 84.5%, and then the cooling crystallization is performed.

The purification method of NMMO in the invention, wherein the mass concentration of NMMO in the lyocell fiber coagulation bath is 56.5% ~ 72.2% by the concentration and the temperature of the cooling crystallization is between 25 °C ~ 40 °C.

The purification method of NMMO in the invention, wherein during the cooling crystallization process, a cooling rate is 1 ~ 2°C/ hour from an initial appearance of crystals to 30°C; the cooling rate is 3 ~ 4°C/ hour between -20°C ~ 30°C.

The purification method of NMMO in the invention, wherein seed crystals are added during the cooling crystallization process, and the amount of the seed crystals added is above 0.01% of the weight of the NMMO in the lyocell fiber coagulation bath.

The purification method of NMMO in the invention, wherein the cooling crystallization includes at least a primary cooling crystallization and a secondary cooling crystallization, the NMMO hydrate crystals and a crystal mother solution are obtained by the primary cooling crystallization, and the crystal mother solution is first concentrated and then subjected to the secondary cooling crystallization.

The purification method of NMMO in the invention, wherein performing the microfiltration to the mixture to obtain microfiltration filtrate; performing ultrafiltration to the microfiltration filtrate to obtain an ultrafiltration trapped concentrate and an ultrafiltration filtrate; performing the cooling crystallization to the ultrafiltration filtrate; wherein, the filter aperture of the microfiltration is 0.5 ~ 5 µm; the ultrafiltration has molecular weight cut off 1000 ~ 100000.

The purification method of NMMO in the invention, wherein performing a first nanofiltration to the ultrafiltration filtrate first to obtain a first nanofiltration trapped concentrate and a first nanofiltration filtrate, and then performing the cooling crystallization to the first nanofiltration filtrate; wherein, the first nanofiltration has molecular weight cut off 300 ~ 1000.

The purification method of NMMO in the invention, wherein performing a second nanofiltration to the first nanofiltration filtrate first to obtain a second nanofiltration trapped concentrate and a second nanofiltration filtrate, and the NMMO is kept in the second nanofiltration trapped concentrate, then performing the cooling crystallization to the second nanofiltration trapped concentrate; wherein, the second nanofiltration has molecular weight cut off 100 ~ 200.

To achieve the above objects, the invention further provides a NMMO hydrate crystal obtained by the purification method.

To achieve the above objects, the invention further provides a purification system of NMMO, which is used to purify NMMO in a lyocell fiber coagulation bath, comprising:
a crystallization device configured to crystallization process of the lyocell fiber coagulation bath;
a control device connected to the crystallization device to control crystallization conditions in the crystallization device.

The purification system of NMMO in the invention, wherein further comprises:
at least one of a microfiltration device, an ultrafiltration device and a nanofiltration device connected to the crystallization device and the control device, and the lyocell fiber coagulation bath flows into at least one of the microfiltration device, the ultrafiltration device and the nanofiltration device for processing to obtain a filtrate and the filtrate flows into the crystallization device for crystallization.

The purification system of NMMO in the invention, wherein further comprises:
a flocculation and sedimentation device, and the lyocell fiber coagulation bath and coagulant aids flow into the flocculation and sedimentation device to obtain a mixture to perform flocculation and sedimentation;
the microfiltration device is connected to the flocculation and sedimentation device so that the mixture flows into the microfiltration device to filter solids and obtain microfiltration filtrate;
the ultrafiltration device is connected to the microfiltration device and the microfiltration filtrate flows into the ultrafiltration device to obtain an ultrafiltration trapped concentrate and an ultrafiltration filtrate;
the nanofiltration device is connected to the ultrafiltration device and the crystallization device respectively, the ultrafiltration filtrate flows into the nanofiltration device to obtain a nanofiltration trapped concentrate and a nanofiltration filtrate, and the NMMO is kept in the nanofiltration trapped concentrate, which flows into the crystallization device for crystallization.

The purification system of NMMO in the invention, wherein the nanofiltration device comprises a first nanofiltration device and a second nanofiltration device;
the first nanofiltration device is connected to the ultrafiltration device, and the ultrafiltration filtrate flows into the first nanofiltration device to obtain a first nanofiltration trapped concentrate and a first nanofiltration filtrate;
the second nanofiltration device is connected to the first nanofiltration device and the crystallization device respectively, the first nanofiltration filtrate flows into the second nanofiltration device to obtain a second nanofiltration trapped concentrate and a second nanofiltration filtrate, and the NMMO is kept in the second nanofiltration trapped concentrate, which flows into the crystallization device for crystallization.

The invention has the following beneficial effects:
The invention adopts the technology of crystallization process or the technology combining membrane separation with crystallization process to purify and recover the NMMO in the lyocell fiber coagulation bath. Comparing to the method for purifying and recovering the NMMO in the lyocell fiber coagulation bath using ion-exchange resin, the invention obtains NMMO crystals with higher purity, which are equal to the purity of commercially available NMMO products and fully reusable for the lyocell production, and the total recovery rate of NMMO is high. The invention generates almost no waste gas, waste water, and solid waste during the purification process and the purification and recovery cost is low.

In addition, the purification process of the invention is simple and suitable for industrial production. The purification process of the invention is an efficient, simple, low-cost and green purification method for NMMO.

### BRIEF DESCRIPTION OF THE DRAWINGS

Figure 1 is a schematic diagram of membrane separation processing device in a purification system of NMMO according to one embodiment of the invention.
Figure 2 is a schematic diagram of crystallization device in a purification system of NMMO according to one embodiment of the invention.
Figure 3 is a HPLC chromatography of the microfiltration filtrate according to embodiment 2 of the invention.
Figure 4 is a HPLC chromatography of the ultrafiltration trapped concentrate according to embodiment 2 of the invention.
Figure 5 is a HPLC chromatography of the ultrafiltration filtrate according to embodiment 2 of the invention.
Figure 6 is a HPLC chromatography of the first nanofiltration filtrate according to embodiment 2 of the invention.
Figure 7 is a HPLC chromatography of the first nanofiltration trapped concentrate according to embodiment 2 of the invention.

### Reference numerals:

101 lyocell fiber coagulation bath
102 coagulant aids
103 ultrafiltration trapped concentrate
104 ultrafiltration filtrate
105 solids
106 microfiltration filtrate
107 first nanofiltration trapped concentrate
108 first nanofiltration filtrate
109 second nanofiltration trapped concentrate
110 second nanofiltration filtrate
111 reverse osmosis trapped concentrate
112 reverse osmosis filtrate
A flocculation and sedimentation device
B microfiltration device
C ultrafiltration device
D first nanofiltration device
E second nanofiltration device
F reverse osmosis device
1 first concentration device
11 condensate
12 first concentrated solution
2 first crystallization device
3 first crystallization separation device
31 crystals from the first crystallization
32 mother solution of the first crystallization
4 second concentration device
41 second concentrated solution
42 condensate
5 second crystallization device
6 second crystallization separation device
61 crystals from the second crystallization
62 mother solution of the second crystallization
7 dissolution device
71 water
72 crystal solution
8 recrystallization device
9 recrystallization separation device
91 recrystallization crystals
92 recrystallization mother solution

### DETAILED DESCRIPTION

Embodiments of the invention are described in detail as follows. The embodiments are implemented under the premise of technical solutions of the invention and detailed implementation methods and processes are provided, but the protection scope of the invention is not limited to the embodiments. Experimental methods for which no specific conditions are specified in the embodiments are generally based on conventional conditions.

The invention provides a purification method of NMMO, which is used to purify NMMO in a lyocell fiber coagulation bath, comprising the following steps: performing cooling crystallization to the lyocell fiber coagulation bath between -20 °C and 78 °C to obtain NMMO hydrate crystals.

Impurities in the lyocell coagulation bath are complex, including a relatively large amount of carbohydrate impurities such as oligosaccharide, polysaccharide, monosaccharide, etc., carbohydrate decomposition products such as furfurals impurities, a small amount of residual flocculant PAM, a certain amount of PG, PG oxides, reaction products of PG and formaldehyde and other impurities, NMMO decomposition products such as N-methylmorpholine, morpholine and other impurities, and also other various unknown impurities from the reaction of NMMO with carbohydrate decomposition products in addition to the NMMO with mass content of 10-25%.

Crystallization purification method is performed by cooling down the temperature and taking advantage of the difference in solubility of each component of the mixture in a solvent, such that the concentration of the component desired to be crystallized and to be purified in the mother solution reaches the supersaturation to achieve a supersaturated solution. The component desired to be crystallized and to be purified is precipitated from the supersaturated solution in crystal form, while impurity components with a relatively low content is left in the mother solution, so as to achieve the effect of separation and purification.

Since transporting pure NMMO solid has difficulty in industry and NMMO aqueous solution is used as raw material in lyocell industry, the NMMO is usually produced as an aqueous solution in this field.

The invention takes advantage of crystal properties of NMMO hydrates and controls the cooling crystallization conditions to combine the NMMO with water at a specific proportion to form hydrate crystals, thereby achieving the goal of separating the NMMO from impurities in aqueous solutions. In other words, the invention obtains NMMO hydrate solids through crystallization, achieving the purpose of purifying the NMMO, and then the NMMO hydrate solids is prepared into an aqueous solution of a required concentration to be reused in the lyocell fiber production.

The NMMO in the lyocell fiber coagulation bath containing the above-mentioned impurities can be purified and recovered directly by the crystallization method. However, the existence of carbohydrates, etc. in the lyocell fiber coagulation bath leads to a relatively high viscosity of the crystallization solution and further negatively affects the crystallization process of the NMMO hydrates and the separation of NMMO hydrate crystals, which makes it difficult to obtain NMMO hydrate crystals in high purity and high yields. In addition, these impurities, especially the carbohydrates, will all remain in the crystal mother solution, resulting in a high viscosity of the crystal mother solution, making it difficult to flow and unable to perform crystallization to the crystal mother solution again to further recover the NMMO. Therefore, before crystalizing and purifying the NMMO in the lyocell fiber coagulation bath, it is better to remove most of the macromolecular impurities such as carbohydrate impurities and PAM in the lyocell fiber coagulation bath in advance.

In one embodiment, the invention first removes the carbohydrates in the lyocell fiber coagulation bath and removes molecules with relatively large molecular mass in the lyocell fiber coagulation bath at the same time, and then performs cooling crystallization. In another embodiment, the invention adopts membrane separation processes (at least one of the microfiltration, ultrafiltration and nanofiltration) to remove cellulose, hemicellulose, oligosaccharides, disaccharides, polysaccharides with more than two saccharides, some monosaccharides and other impurities in the lyocell fiber coagulation bath. Meanwhile, the method of membrane separation processes can also remove all of the flocculant PAM, some small molecules with relatively large molecular mass such as reaction products of PG and formaldehyde, inorganic salts with two or more valence and other impurities. Performing the membrane separation processes followed by the cooling crystallization between -20 °C and 78 °C to the lyocell fiber coagulation bath can avoids effects of carbohydrates and other impurities in the lyocell fiber coagulation bath on the crystallization, which can further improve the purity and yield of the NMMO hydrate crystals.

In one embodiment, the invention mixes the lyocell fiber coagulation bath and coagulant aids to obtain a mixture to perform flocculation and sedimentation; then performs at least one of the microfiltration, ultrafiltration and nanofiltration to the mixture.

The flocculation and sedimentation enable insoluble solids and colloid to flocculate and the following membrane separation processes can further separate the solids which still exist in the coagulation bath.

In one embodiment, performing the microfiltration to the mixture first to filtrate the solids and obtain a microfiltration filtrate; then performing ultrafiltration to the microfiltration filtrate to obtain an ultrafiltration trapped concentrate and an ultrafiltration filtrate. In another embodiment, the filter aperture of the microfiltration is 0.5 ~ 5µm and the ultrafiltration membrane has molecular weight cut off 1000 ~ 100000. In this method, the microfiltration can remove the solids in the mixture and the ultrafiltration can remove macromolecular substance soluble in the mixture, and the step-by-step process can improve the efficiency of removing impurities and reduce the loading of the ultrafiltration.

The content of NMMO in the ultrafiltration trapped concentrate and the ultrafiltration filtrate of the invention is essentially the same, for example 150 ~ 250 g/l. To improve the recovery efficiency of the NMMO, in one embodiment, performing flocculation and sedimentation again when the ultrafiltration trapped concentrate is recycled back into the lyocell fiber coagulation bath.

In one embodiment, performing the nanofiltration to the ultrafiltration filtrate to obtain a nanofiltration trapped concentrate and a nanofiltration filtrate, while the NMMO is kept in the nanofiltration trapped concentrate.

In another embodiment, the nanofiltration includes a first nanofiltration and a second nanofiltration, and the ultrafiltration filtrate undergoes the first nanofiltration to obtain a first nanofiltration trapped concentrate and a first nanofiltration filtrate; the first nanofiltration membrane has molecular weight cut off 300 ~ 1000 for instance, which means the first nanofiltration can trap the substances with a molecular weight greater than or equal to 300 ~ 1000, such as substances with molecular weight greater than or equal to 300, 400, 500, 700, 900, etc. In this way, first nanofiltration membranes keeps polyvalent ions, chromophore impurities, metal complexes, polysaccharide and other carbohydrates in the first nanofiltration trapped concentrate and the target recoveries, NMMO, and monovalent salts enter the first nanofiltration filtrate allowing further purification of NMMO. The first nanofiltration filtrate undergoes the second nanofiltration to obtain a second nanofiltration trapped concentrate and a second nanofiltration filtrate, while the NMMO is kept in the second nanofiltration trapped concentrate ; the second nanofiltration membrane has molecular weight cut off 100 ~ 200, which means the second nanofiltration can trap the substances with a molecular weight greater than or equal to 100 ~ 200, such as substances with molecular weight greater than or equal to 100, 150, 200, etc. In this way, the target recoveries, NMMO, is kept in the second nanofiltration trapped concentrate, and the monovalent salts and the solvent water enter the second nanofiltration filtrate allowing further purification and preliminary concentration of the NMMO.

The content of NMMO in the first nanofiltration trapped concentrate and the first nanofiltration filtrate is essentially the same, for example 150 ~ 250 g/l.

To improve the recovery efficiency of the NMMO and reduce the loss of the NMMO in the process, the first nanofiltration also includes a first diafiltration of the first nanofiltration trapped concentrate to obtain a first nanofiltration diafiltrate, where the first nanofiltration trapped concentrate is continuously supplied with water for at least one time, for example can be 2 times, 4 times, 6 times, 8 times, etc. The number of the first diafiltration is mainly determined by the content of the NMMO in the first nanofiltration trapped concentrate after the first diafiltration. When the desired NMMO content in the first nanofiltration trapped concentrate after the first diafiltration is reached (0 ~ 10g/l, for instance) the first diafiltration stops and the first nanofiltration trapped concentrate is discharged outside. At mean time, combining all the obtained first nanofiltration diafiltrates together and the content of the NMMO inside is normally 10 ~ 100g/l. Then the mixture of the obtained first nanofiltration diafiltrate and the first nanofiltration filtrate undergoes the second nanofiltration together.

The content of NMMO in the second nanofiltration trapped concentrate is for example 150 ~ 250g/l, the electrical conductivity is 2 ~ 500µs and the content of NMMO in the second nanofiltration filtrate is for example 0 ~ 5g/l, the electrical conductivity is 2 ~ 300µs. At this time, a certain amount of the monovalent salts still remains in the second nanofiltration trapped concentrate. To further purify the NMMO and remove the impurities in the NMMO, the second nanofiltration also includes a second diafiltration of the second nanofiltration trapped concentrate to obtain a second nanofiltration diafiltrate, where the second nanofiltration trapped concentrate is continuously supplied with water for at least one time, for example can be 2 times, 4 times, 6 times, 8 times, etc. The number of the second diafiltration is mainly determined by the content of the monovalent salts in the second nanofiltration trapped concentrate, for instance when the content of the NMMO in the second nanofiltration trapped concentrate after the second diafiltration is 150 ~ 250g/l and the electrical conductivity is 1 ~ 5µs while the content of NMMO in the second nanofiltration diafiltrate is 0 ~ 5g/l, the electrical conductivity is 1 ~ 1000µs, the second diafiltration stops.

In the above process, the second nanofiltration filtrate and the second nanofiltration diafiltrate can undergo reverse osmosis membrane process to obtain a reverse osmosis trapped concentrate containing impurities of monovalent salts and a reverse osmosis filtrate. The reverse osmosis filtrate is used as water for the above first diafiltration and the second diafiltration to conserve water resources and reduce the production of wastewater; the reverse osmosis trapped concentrate is discharged outside or performed with further process.

In one embodiment, the concentration ratio of the reverse osmosis membrane process is determined in accordance with the electrical conductivity of the reverse osmosis filtrate in the invention, for example the electrical conductivity of the reverse osmosis filtrate is less than 1µs.

In one embodiment, various impurities with low molecular mass including PG, PG oxides remains in the second nanofiltration trapped concentrate obtained by the second nanofiltration, and results of HPLC analysis shows that the second nanofiltration trapped concentrate still contains a certain amount of impurities such as PG and PG oxidation products together with other multiple unknown impurities. The second nanofiltration trapped concentrate needs further purification for better use in the lyocell fiber production. Hence, the second nanofiltration trapped concentrate in the invention needs a further crystallization process. In another embodiment, the second nanofiltration trapped concentrate in the invention is performed with a crystallization process without the second diafiltration.

The invention doesn't specifically limit that the mixture must be performed with all steps of the microfiltration, the ultrafiltration, the first nanofiltration and the second nanofiltration before the crystallization. For instance, performing the microfiltration to the mixture first and then performing the cooling crystallization to the microfiltration filtrate; performing the microfiltration and the ultrafiltration to the mixture first and then performing the cooling crystallization to the ultrafiltration filtrate; performing the microfiltration, the ultrafiltration, and the nanofiltration to the mixture first and then performing the cooling crystallization to the nanofiltration trapped concentrate ; performing the microfiltration, the ultrafiltration, the first nanofiltration and the second nanofiltration to the mixture first and then performing the cooling crystallization to the second nanofiltration trapped concentrate .

In one embodiment, the liquid which is going to be crystallized (such as the lyocell fiber coagulation bath, the microfiltration filtrate, the ultrafiltration filtrate, the first nanofiltration trapped concentrate or the second nanofiltration trapped concentrate) is concentrated before the cooling crystallization in the invention, until the mass concentration of the NMMO in the liquid is 56.5% ~ 84.5% and the crystallization efficiency is improved thereby. In another embodiment, the method of concentration is evaporation, such as vacuum evaporation, reduced pressure evaporation, atmospheric evaporation, etc.

In one embodiment, the invention performs the concentration to the liquid above stated to be crystallized and the mass content of the NMMO in the obtained concentrated solution is 56.5% ~ 72.2%. The cooling crystallization to the concentrated solution is carried out at temperatures between -20 ~ 39 °C, and NMMO hydrate crystals, 2NMMO·5H₂O (i.e. NMMO·2.5H₂O), are obtained; when the mass content of the NMMO in the obtained concentrated solution is 72.2% ~ 84.5% (excluding 72.2%), performing cooling crystallization to the concentrated solution at temperatures between 39 ~ 78°C (excluding 39°C), so that the obtained crystalline NMMO is NMMO·H₂O. In other words, the different concentrations of NMMO in the concentrated solution determine the different crystallization temperatures, and further resulting in different NMMO hydrate crystals.

In one embodiment, consider the cost of industrial purification and operation convenience, the liquid to be crystallized is concentrated to NMMO mass concentration of 56.5% ~ 72.2% or 66.5% ~ 72%, and the cooling crystallization of the concentrated solution can be performed at a temperature range between 25 ~ 40°C or 25 ~ 36°C (the final crystallization temperature is 25°C and above) in the invention. Under the optimal crystallization conditions, high purity of 2NMMO·5H₂O crystal with a crystallization yield of more than 60% can be obtained after first crystallization; the higher the concentration of the NMMO in the liquid before the crystallization, the lower the final crystallization temperature and the higher the obtained NMMO crystallization yield.

Performing the crystallization under the optimal crystallization conditions, different conditions of the crystallization process, such as crystallization yields, cooling rates, and additions of seed crystals, will affect the degree of supersaturation of the metastable state during the crystallization and therefore affect the purity of the NMMO in crystals.

In one embodiment, seed crystals are added during crystallization process and an amount of the seed crystals added is above 0.01%, which is preferably between 0.1% ~ 0.3%, of the weight of the NMMO in the liquid to be crystallized. In cooling crystallization, seed crystal addition that induces crystallization can effectively reduce the degree of supersaturation of NMMO in solution, and resulting in crystals with higher NMMO purity.

In one embodiment, the seed crystals added in the invention are small particles of broken crystals with high purity, which are crushed and have a particle size as uniform as possible, and the size of the particles for example ranging from 0.01 to 0.1mm. Seed crystals with relatively large particles or insufficient addition of the seed crystals may lead to a decrease in the purity of NMMO crystal, and the obtained NMMO crystal have an uneven distribution of particle size; excessive addition of the seed crystals may lead to relatively small particles of the obtained NMMO crystal. In addition, the seed crystals should not be added too early, otherwise, the temperature of the solution may be too high, resulting in the possibility to dissolve the crystals due to the concentration of NMMO hydrates in the solution lower than NMMO hydrate saturation solubility and the significance of the seed crystal addition will be lost; on the other hand, if the seed crystals are added too late, the temperature of the solution may be too low and the degree of supersaturation of the solution may be too high, which will reduce the actual effect of the seed crystal addition. The timing of adding the seed crystals is generally determined by the NMMO content in the liquid to be crystallized and the initial cooling temperature. In one embodiment, seed crystals are added when the solution is cooled to 30-37 °C.

In one embodiment, the seed crystals of the invention are soaked in a high-purity NMMO saturated aqueous solution for at least 1 hour before being added to the crystallization system, which can improve crystallization effect and ensure crystals precipitated in the crystallization system have a uniform size.

In one embodiment, during the cooling crystallization process of the invention, from initial appearance of crystals in the crystallization system till the temperature of the crystallization system is cooled down to 30°C, the cooling rate is controlled at 1-2 °C/hour, and after the temperature of the crystallization system drops below 30 °C (for example, at -20-30°C), the cooling rate can be controlled at 3-4 °C/hour. In this way, crystal purity can be guaranteed. This is because the cooling rate directly determines the crystallization rate in the cooling crystallization. When the crystallization system is in a relatively high temperature range, the solubility curve of NMMO hydrates varies greatly. Therefore, in the initial stage of crystallization after the crystals begin to appear, the cooling rate of the solution system should not be too fast, and the growth rate of the crystals should be limited, otherwise it will lead to a high degree of supersaturation of the solution and affect the crystal purity.

In one embodiment, after the cooling crystallization system reaches the crystallization termination temperature, the crystallization solution is quickly passed through a Buchner funnel for vacuum filtration, and the crystal mother solution is discharged from the filter bottle. The crystals are rinsed with a high-purity NMMO aqueous solution with a concentration of 59.5% to obtain the NMMO crystal, which are weighed and analyzed for content.

First crystallization can obtain NMMO hydrate crystals with very high purity and the NMMO hydrate crystals are mixed with water to prepare an NMMO aqueous solution with a mass concentration of 19.8%, which is colorless and transparent, and the electrical conductivity can reach 14.9µs/cm. To obtain a NMMO with higher purity, crystals obtained by the first crystallization are mixed with water to prepare a NMMO aqueous solution with a certain concentration for secondary crystallization (recrystallization). Crystals obtained by the secondary crystallization are analyzed and detected by liquid chromatography, and the result shows that various organic impurities present in the original lyocell fiber coagulation bath are not detectable in the NMMO crystal. The crystals obtained by the second crystallization are mixed with water to prepare a NMMO aqueous solution with a mass content of 50% and the electrical conductivity of the NMMO aqueous solution may decrease to less than 12.5µs/cm. Analysis of the total carbohydrate content in the crystal mother solution shows that the carbohydrate content is very low, which indicates that most of the carbohydrate impurities in the lyocell fiber coagulation can be removed after the membrane separation process.

Macromolecular impurities, such as oligosaccharides, etc., affecting the viscosity of the coagulation bath are removed from the lyocell fiber coagulation bath after the membrane separation process, which greatly reduces the viscosity of the crystal mother solution so that the crystal mother solution can achieve multiple concentration and crystallization. In an embodiment of the invention, evaporating the mother solution produced by the first crystallization through vacuum heating to the NMMO mass concentration of 69 ~ 72%, and performing the cooling crystallization under the optimal crystallization condition, which has a crystallization yield of 80 ~ 85%. The purity of the secondary crystalline crystals is slightly lower than the first crystallization and the secondary crystalline crystals can return to dissolve in raw materials of the coagulation bath. The total NMMO recovery rate of the first crystallization and the second crystallization can reach 96%. The viscosity of the mother solution of the second crystallization is still not high, and the mother solution can continue to be concentrated and crystallized to further improve the total recovery rate of the NMMO.

In one specific embodiment, the crystallization method in the invention includes: the lyocell fiber coagulation bath with a NMMO mass concentration of 66.5-72% at a temperature of about 40°C after the membrane separation process is cooled down to a temperature at which the seed crystals will not dissolve after being added (the temperature is known as the temperature of the initial appearance of crystals), and then a small amount of seed crystals is added. The seed crystals are preferably broken crystals of NMMO·2.5H₂O in the form of small particle, which are pre-soaked in a high-purity NMMO saturated solution. The broken crystals in the form of small particle are soaked in the NMMO saturated solution for at least 1 hour, and the amount of seed crystals added is 0-1% of the mass of the NMMO in the coagulation bath, which is preferably 0.1-0.3%. After adding seed crystals, continuing to cool down the lyocell fiber coagulation bath slowly. The cooling rate is controlled at 1 - 2°C/ hour from the temperature of the initial appearance of crystals to 30°C. When the temperature drops below 30 °C, the cooling rate can be appropriately accelerated to 3-4 °C/hour, and the crystallization termination temperature is 25 °C. Crystals in the form of large particle with an even distribution of particle size and low impurity content can be obtained under this crystallization condition. After rapid vacuum filtration of the crystal solution, the mother solution is collected and weighed. The crystals are rinsed with a high-purity NMMO aqueous solution with a mass concentration of 59% and the obtained crystals are crystals from the first crystallization, which are collected to be weighed and analyzed for crystal purity. An aqueous solution with NMMO mass concentration of 19.8% is prepared by adding water to the crystals, and the total carbohydrate content, total impurity content, the electrical conductivity, content of copper and iron metal ions, etc. are detected. If a NMMO with higher purity is required, the crystal from the first crystallization is first dissolved by adding water and then undergoes crystallization again (the secondary crystallization). The process conditions of the secondary crystallization are similar to those of the first crystallization. Crystals from the secondary crystallization are collected to be weighed and analyzed for crystal purity. Crystallization separation may adopt the method of batch crystallization or continuous crystallization.

Therefore, the invention provides a method for purifying the NMMO in the lyocell fiber coagulation bath by crystallization methods. The method first adopts membrane separation process to remove most of the macromolecular impurities such as carbohydrates, PAM, etc. in the lyocell fiber coagulation bath that affecting the viscosity of the coagulation bath. Elimination of undesirable effects of these macromolecular impurities on the crystallization can improve the recovery rate of the NMMO. Then concentrating the lyocell fiber coagulation bath after the membrane separation process by heating evaporation under reduced pressure until the NMMO mass content is 56.5 ~ 84.5% and NMMO hydrate crystals with high purity can be obtained by the crystallization. Within the above range of NMMO mass concentration, the corresponding cooling crystallization can be run at the temperature between -20 ~ 78 °C. Consider the cost of industrial purification and operation convenience, the mass concentration of the NMMO in the coagulation bath after concentration is preferably controlled at 66.5 ~ 72%, thus the crystallization and purification can be performed in a very suitable temperature range of 25 ~ 35°C, and high purity of 2NMMO·5H₂O crystals (the NMMO content in the crystals is 72.2%) with a crystallization yield of more than 60% can be obtained.

Different from existing NMMO purification and recovery methods using ion-exchange resins, the invention does not use ion-exchange resins. In a result, environmental issues such as a significant amount of wastewater with high concentration of salt and COD content produced from the regeneration of the ion-exchange resins, spent ion-exchange resins and other hazardous wastes don't exist. The purity of the NMMO, which is purified and recovered according to the crystallization method provided by the invention, is very high and almost all impurities, including many non-ionic impurities that cannot be removed by the method of ion-exchange resins, in the lyocell coagulation bath can be removed. The purity of the obtained NMMO is much higher than that of NMMO purified by the current ion-exchange resin method. The invention has a simple purification process, low purification cost, and high yield of NMMO and the invention is an efficient, simple, low-cost and green purification method for NMMO.

The invention further provides a purification system of NMMO, which is used to purify NMMO in a lyocell fiber coagulation bath, comprising:
a crystallization device configured to crystallization process of the lyocell fiber coagulation bath;
a control device connected to the crystallization device to control crystallization conditions in the crystallization device.

In one embodiment, the purification system of NMMO in the invention further comprises:
at least one of a microfiltration device, an ultrafiltration device and a nanofiltration device connected to the crystallization device and the control device, and the lyocell fiber coagulation bath flows into at least one of the microfiltration device, the ultrafiltration device and the nanofiltration device for processing to obtain a filtrate and the filtrate flows into the crystallization device for crystallization.

In another embodiment, the purification system of NMMO in the invention further comprises:
a flocculation and sedimentation device, and the lyocell fiber coagulation bath and coagulant aids flow into the flocculation and sedimentation device to obtain a mixture to perform flocculation and sedimentation;
the microfiltration device is connected to the flocculation and sedimentation device so that the mixture flows into the microfiltration device to filter solids and obtain microfiltration filtrate;
the ultrafiltration device is connected to the microfiltration device and the microfiltration filtrate flows into the ultrafiltration device for ultrafiltration to obtain an ultrafiltration trapped concentrate and an ultrafiltration filtrate;
the nanofiltration device is connected to the ultrafiltration device and the crystallization device respectively, the ultrafiltration filtrate flows into the nanofiltration device for nanofiltration to obtain a nanofiltration trapped concentrate and a nanofiltration filtrate, and the NMMO is kept in the nanofiltration trapped concentrate, which flows into the crystallization device for crystallization.

In another embodiment, the nanofiltration device comprises a first nanofiltration device and a second nanofiltration device;
the first nanofiltration device is connected to the ultrafiltration device, and the ultrafiltration filtrate flows into the first nanofiltration device for first nanofiltration to obtain a first nanofiltration trapped concentrate and a first nanofiltration filtrate;
the second nanofiltration device is connected to the first nanofiltration device and the crystallization device respectively, the first nanofiltration filtrate flows into the second nanofiltration device for second nanofiltration to obtain a second nanofiltration trapped concentrate and a second nanofiltration filtrate, and the NMMO is kept in the second nanofiltration trapped concentrate, which flows into the crystallization device for crystallization.

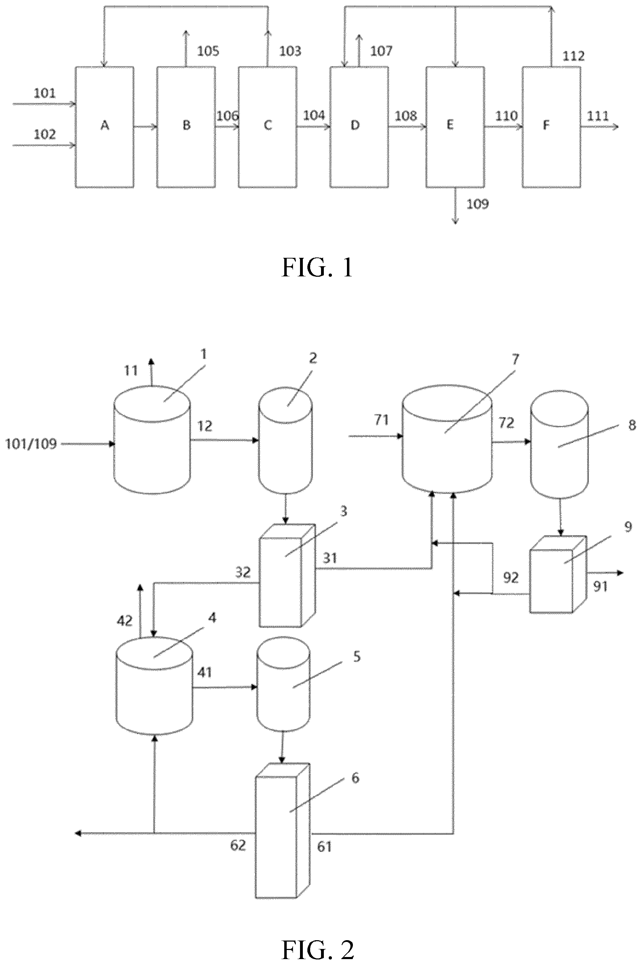

In one specific embodiment, the membrane separation processing device in the purification system of NMMO in the invention is shown in Figure 1. The lyocell fiber coagulation bath 101 and the coagulant aids 102 flows into the flocculation and sedimentation device A to obtain the mixture by mixing and to perform flocculation and sedimentation; the microfiltration device B is connected to the flocculation and sedimentation device A so that the mixture flows into the microfiltration device B for microfiltration to filter solids 105 and obtain the microfiltration filtrate 106; the ultrafiltration device C is connected to the microfiltration device B and the microfiltration filtrate 106 flows into the ultrafiltration device C for ultrafiltration to obtain the ultrafiltration trapped concentrate 103 and the ultrafiltration filtrate 104. To improve recovery efficiency of the NMMO, the ultrafiltration trapped concentrate 103 in the invention can be recycled back to the flocculation and sedimentation device A to mix with the lyocell fiber coagulation bath 101 and perform the flocculation and sedimentation again.

The first nanofiltration device D is connected to the ultrafiltration device C so that the ultrafiltration filtrate 104 is transported to the first nanofiltration device D for first nanofiltration to obtain the first nanofiltration trapped concentrate 107 and the first nanofiltration filtrate 108; the second nanofiltration device E is connected to the first nanofiltration device D so that the first nanofiltration filtrate 108 is transported to the second nanofiltration device E for second nanofiltration to obtain the second nanofiltration trapped concentrate 109 and the second nanofiltration filtrate 110.

Reverse osmosis device F is connected to the second nanofiltration device E so that the second nanofiltration filtrate 110 is transported to the reverse osmosis device F to perform reverse osmosis membrane process, and the reverse osmosis trapped concentrate 111 containing impurities of monovalent salts and the reverse osmosis filtrate 112 are obtained.

The reverse osmosis filtrate 112 can be recycled back to the first nanofiltration device D and the second nanofiltration device E to be used as water for the first diafiltration and the second diafiltration to conserve water resources and reduce the production of wastewater; the reverse osmosis trapped concentrate 111 is discharged outside or performed with further process.

The invention doesn't specifically limit that the membrane separation processing device in the purification system of NMMO includes each of the above units. In one embodiment, the membrane separation processing device may not include the above microfiltration device B; in another embodiment, the membrane separation processing device may not include the above first nanofiltration device D and the second nanofiltration device E. The above units can be adjusted as needed.

In one specific embodiment, the crystallization device in the purification system of NMMO in the invention is shown in Figure 2. A first concentration device 1 may be connected to the second nanofiltration device E and a first crystallization device 2, and the second nanofiltration trapped concentrate 109 (or the lyocell fiber coagulation bath 101) flows into the first concentration device 1 for concentration process. The first concentration device 1, for example, is an evaporation device. Condensate 11 flows out from the top of the first concentration device 1, and an obtained first concentrated solution 12 flows into the first crystallization device 2 for crystallization process. The crystallization conditions have been explained in detail above and no further elaboration is made here.

The first crystallization device 2 is connected to a first crystallization separation device 3, and a mixture obtained from the crystallization in the first crystallization device 2 flows into the first crystallization separation device 3 for solid-liquid separation to obtain crystals from the first crystallization 31 and a mother solution of the first crystallization 32.

In another embodiment, the first crystallization separation device 3 is connected to a second concentration device 4 such that the mother solution of the first crystallization 32 flows into the second concentration device 4 for concentration process, and a second concentrated solution 41 and a condensate 42 are obtained; the second concentration device 4 is connected to a second crystallization device 5, and the second concentrated solution 41 flows into the second crystallization device 5 for second crystallization process. In this way, the yield of the NMMO can be improved.

The second crystallization device 5 is connected to a second crystallization separation device 6, and a mixture obtained from the crystallization in the second crystallization device 5 flows into the second crystallization separation device 6 for solid-liquid separation to obtain crystals from the second crystallization 61 and a mother solution of the second crystallization 62. In one embodiment, the second crystallization separation device 6 is also connected to the second concentration device 4, so that the mother solution of the second crystallization 62 can be recycled back to the second concentration device 4 for concentration process, and then go back to the crystallization process again to further improve the yield of the NMMO. Due to the low content of the NMMO in the mother solution of the second crystallization 62, the mother solution of the second crystallization 62 can be directly discharged outside.

In one embodiment, the purification system of NMMO of the invention further includes a dissolution device 7, which is connected to the first crystallization separation device 3 and the second crystallization separation device 6 respectively such that the crystals from the first crystallization 31 and the crystals from the second crystallization 61 are transported to the dissolution device 7, and are mixed with input water 71 to obtain a crystal solution 72.

The dissolution device 7 is connected to a recrystallization device 8, which is also connected to a recrystallization separation device 9. The crystal solution 72 is transported to the recrystallization device 8 for recrystallization process, which can further improve the purity of the NMMO. A mixture obtained from the crystallization in the recrystallization device 8 is transported to the recrystallization separation device 9 to obtain recrystallization crystals 91 and a recrystallization mother solution 92. The sample of the recrystallization crystals 91 is analyzed with liquid chromatography and the analysis results show that various organic impurities present in the original lyocell fiber coagulation bath are not detectable in the NMMO crystal. The recrystallization crystals 91 are mixed with water to prepare an NMMO aqueous solution with a mass content of 50% and the electrical conductivity of the NMMO aqueous solution may decrease to less than 2.5µs/cm.

In one embodiment, the recrystallization separation device 9 is also connected to the first crystallization separation device 3 and the second crystallization separation device 6 respectively such that the recrystallization mother solution 92 is used for washing the crystals from the first crystallization 31 and the crystals from the second crystallization 61, which reducing the mother solution adsorbed on the surface of the crystals from the first crystallization 31 and the crystals from the second crystallization 61, and further removing impurities embedded in the crystals from the first crystallization 31 and the crystals from the second crystallization 61.

In one embodiment, the purification system of NMMO of the invention is used in industrial production. The purification system also includes a control device (not shown in the figure), which is electrically connected to one or more of the first concentration device 1, the first crystallization device 2, the first crystallization separation device 3, the second concentration device 4, the second crystallization device 5, the second crystallization separation device 6, the dissolution device 7, the recrystallization device 8, and the recrystallization separation device 9 respectively, to control process parameters of the crystallization process.

Hereinafter, the technical solution of the invention is further described in detail by specific embodiments. Unspecified '%' generally refers to percentage of mass hereinafter.

The detection method of the invention:
The detection of iron ion content and copper ion content: instrument is Thermo Fisher ICP-MS.

The detection of total carbohydrate content: using the analytical method for total carbohydrate disclosed in the `Study on Lyocell fiber from a cheap pulp with high hemicellulose content', which is a PhD dissertation of the Donghua University written by Huiru Chen.

The detection of organic impurities, PG and PG oxides: using liquid chromatography (HPLC) analysis, and chromatographic column is: AichromBond-1, C18, 5 µm 4.6x150mm; mobile phase: 0.2% phosphate buffer, pH = 2.9; flow rate of mobile phase: 1 ml/min; column temperature: 35 °C; UV detection wavelength: 200nm and 220nm, the detections are performed separately; sample size: 20µl; detection time: 30 minutes.

The detection of purity of the NMMO crystal (residual rate of impurity): using the residual rate of impurity to show the purity of the obtained NMMO hydrate crystals. The analytical method to detect the purity is: adjusting the NMMO mass concentration in raw materials of the lyocell fiber coagulation bath to 19.8% to obtain a raw material solution of the lyocell fiber coagulation bath; adding water to the NMMO hydrate crystals obtained by the crystallization to prepare an aqueous solution with NMMO mass concentration of 19.8% and obtaining a crystal solution; using HPLC to analyze and detect the raw material solution of the lyocell fiber coagulation bath and the crystal solution, and UV detection wavelengths are 200nm and 220nm respectively. At each detection wavelength, the ratio of the sum of area integrals of all impurity peaks in the chromatograms of the crystal solution and the sum of area integrals of all impurity peaks in the chromatograms of the raw material solution of the lyocell fiber coagulation bath is calculated separately, and the residual rate of crystal impurity at each UV detection wavelength is obtained thereby.

The residual rate of crystal impurity = (the sum of area integrals of all impurity peaks in the chromatograms of the NMMO crystal solution) ÷ (the sum of area integrals of all impurity peaks in the chromatograms of the raw material solution of the lyocell fiber coagulation bath)

All the samples in the embodiments of the invention below are analyzed and detected in accordance with the above method.

### Embodiment 1.

Purification effects of a lyocell fiber coagulation bath, which is directly performed with the cooling crystallization without any combined carbohydrate removal membrane separation process, are examined.

Properties of the lyocell fiber coagulation bath without any combined carbohydrate removal membrane separation preprocess are as follows:
Mass content of the NMMO: 19.8%
Total carbohydrate content: 502 ppm
Electrically conductivity: 381µs/cm
Content of N-methylmorpholine: 140 ppm
Content of Morpholine: 36 ppm
Content of iron ions: 1.89 ppm
Content of copper ions: 1.23 ppm
Turbidity: 15 NTU
Chromaticity: 957

Concentrating the above original lyocell fiber coagulation baths by heating evaporation under reduced pressure until the NMMO mass concentration is 68.0% and 69.9% and performing the cooling crystallization separately. The concentrated solution of the original coagulation baths at each concentration starts to cool down at 37°C. When the solution with NMMO concentration of 68.0% is cooled down to 34.7°C while the solution with NMMO concentration of 69.9% is cooled down to 35.5°C separately, seed crystals pre-soaked in NMMO saturated solution are added and the amount of the seed crystals added is 0.15% of the NMMO mass in the coagulation bath. After the seed crystals are added, the cooling rate is controlled at 1-2°C/ hour until the temperature drops to 30°C, and then the cooling rate is accelerated to 3-4°C/ hour. When the temperature drops to 25°C, stop the crystallization. Due to the high viscosity of the crystallization system in crystallization process, it is difficult to stir in the later stage of the crystallization and the fluidity of the whole crystallization system after the end of the crystallization is poor, which makes it difficult to separate the mother solution and crystals by vacuum filtration. Hence, it is very difficult to obtain a relatively high NMMO crystallization yield. A small amount of the crystals and mother solution is placed in the Buchner funnel separately and is soaked and rinsed with a significant amount of high-purity NMMO solution with a mass concentration of 59% for 3 times and then the crystal purity (residual rate of impurity) is analyzed and detected.

The obtained crystals and crystal mother solution are added with water separately to prepare an aqueous solution with NMMO mass concentration of 19.8%, and then undergo multiple analyses and detections. The results show that the crystallization can effectively remove various organic impurities contained in the coagulation bath and the NMMO crystal obtained has a very high purity. In addition, the crystallization can also remove metal and non-metal ions contained in the coagulation bath, such that the electrical conductivity of the NMMO crystal is significantly decreased. Therefore, the crystallization can be used as a method and mean for purification and recovery of NMMO in the lyocell coagulation bath. However, the lyocell coagulation bath without any combined carbohydrate removal membrane separation preprocess contains a large amount of carbohydrates, resulting in a very high viscosity of the crystal mother solution which can barely flow. When the crystallization yield is relatively high, crystals and crystal mother solution are difficult to be separated and the viscous crystal mother solution are not able to be crystallized again. Table 1 below shows data of the crystallization and purification of the NMMO in the lyocell fiber coagulation bath without combined membrane separation preprocess.

**Table 1. Data of the crystallization and purification of the NMMO in the lyocell fiber coagulation bath without combined membrane separation preprocess**

| Initial concentration of crystallization solution | Residual rate of impurity (detection at UV wavelength of 200nm) | Residual rate of impurity (detection at UV wavelength of 220nm) | Carbohydrate content (ppm) | Electrical conductivity (µs/cm) |
|---|---|---|---|---|
| Crystalline crystals with NMMO concentration of 68.0% | 0.12% | 0.06% | Unable to detect the presence of carbohydrate | 44.2 |
| Crystal mother solution with NMMO concentration of 68.0% | - | - | 1537 | 1105 |
| Crystalline crystals with NMMO concentration of 69.9% | 0.15% | 0.07% | Unable to detect the presence of carbohydrate | 52.6 |
| Crystal mother solution with NMMO concentration of 69.9% | - | - | 1760 | 1271 |

As shown in table 1, the crystallization can obtain NMMO hydrate crystals with very high purity and separate them from various impurities contained in the lyocell fiber coagulation bath. Although the high content of carbohydrates in the system leads to a high viscosity of the system, difficulty in crystallization, and a relatively small crystallization particle, the impurity content in the obtained crystals is very low. It is mainly due to that the structure of the impurities in the coagulation bath is very different from the NMMO, leading to the fact that these impurities are not easy to enter or being embedded in the crystalline NMMO. Furthermore, the electrical conductivity of the aqueous solution with NMMO concentration of 19.8% prepared by the crystallization is also greatly reduced. However, the crystal purity decreases slightly as the increase in the crystallization yield.

At the same time, the embodiment demonstrates that after the concentration and crystallization of the lyocell coagulation bath without the combined membrane separation process, the viscosity of the crystal mother solution is very high, the separation of the crystals from the crystal mother solution is difficult, the crystal mother solution is not able to further be concentrated and crystallized, and the NMMO recovery rate is relatively low.

### Embodiment 2

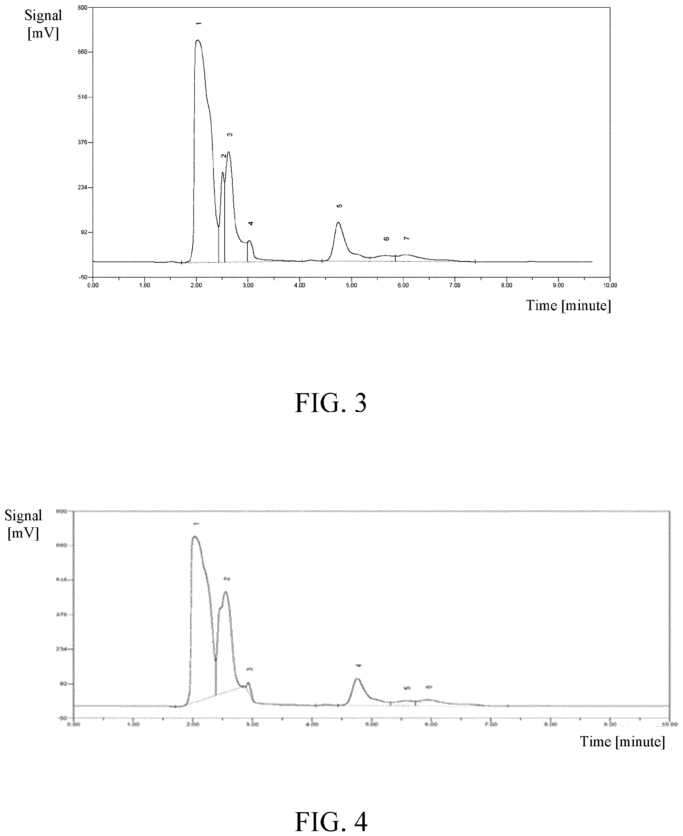

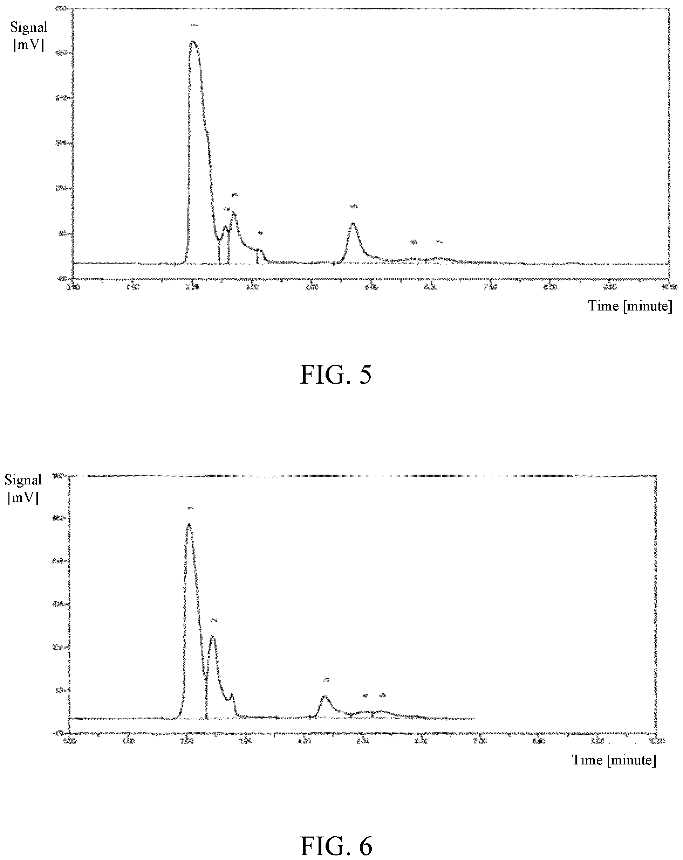

Step 1, mixing the lyocell fiber coagulation bath with coagulant aids of acrylamide to perform flocculation and sedimentation;
Step 2, performing microfiltration, which has a filter aperture of 0.5 ~ 5µm, to the mixture obtained in step 1 to trap solids and obtain a microfiltration filtrate; the HPLC chromatogram of the obtained microfiltration filtrate is shown in Figure 3, wherein peak 1 is the characteristic peak of the NMMO and the others are impurity peaks.
Step 3, performing ultrafiltration with molecular weight cut off 10000 ~ 30000 Dalton to the microfiltration filtrate to trap solids with small particle size, colloid, and macromolecular impurities. The obtained ultrafiltration trapped concentrate is 5% of the volume of the microfiltration filtrate and the ultrafiltration trapped concentrate returns to the step 1 for flocculation again. The HPLC chromatogram of the ultrafiltration trapped concentrate is shown in Figure 4 and the HPLC chromatogram of the ultrafiltration filtrate is shown in Figure 5, wherein peak 1 is the characteristic peak of the NMMO. Comparison of Figure 5 and Figure 3 shows that the purity of the NMMO in the ultrafiltration filtrate is improved.
Step 4, performing the first nanofiltration with molecular weight cut off 500 ~ 800 Dalton to the ultrafiltration filtrate, so that the NMMO and monovalent salts enter the first nanofiltration filtrate while the impurities with medium molecular weight such as chromophore impurities, polysaccharides, metal complexes, high valence ions, etc. are trapped and a first nanofiltration trapped concentrate with concentration ratio of 20 times is obtained; The first nanofiltration trapped concentrate is supplied with water that has 17 times the volume of the first nanofiltration trapped concentrate and the NMMO in the first nanofiltration trapped concentrate is diafiltrated to the first nanofiltration diafiltrate. The NMMO content in the first nanofiltration trapped concentrate is reduced to less than 1g/l and the first nanofiltration trapped concentrate is discharged outside.

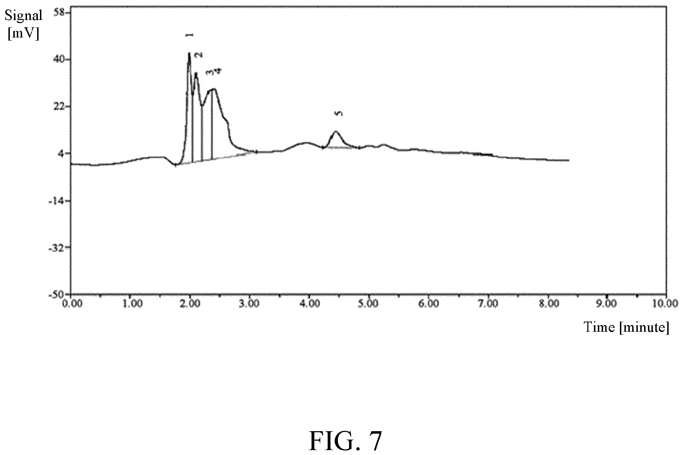

The HPLC chromatogram of the obtained first nanofiltration filtrate is shown in Figure 6, and the HPLC chromatogram of the first nanofiltration trapped concentrate after the diafiltration process is shown in Figure 7, wherein peak 1 is the characteristic peak of the NMMO. As shown in Figure 7, the amount of NMMO in the first nanofiltration trapped concentrate is small, meaning that not too much NMMO is lost in the first nanofiltration step and showing that the diafiltration process can further improve the recovery efficiency of the NMMO. Comparison of Figure 6 and Figure 5 shows that the purity of the NMMO in the first nanofiltration filtrate is improved but a small amount of impurities still presents in the first nanofiltration filtrate.

Step 5, performing the second nanofiltration with molecular weight cut off 300 Dalton to the mixture of first nanofiltration filtrate and first nanofiltration diafiltrate, so that a certain amount of the monovalent salts enters the second nanofiltration filtrate while the NMMO is trapped by the nanofiltration membrane; when the second nanofiltration trapped concentrate is condensed to 0.2 times , the second nanofiltration trapped concentrate is supplied with water that has 10 times the volume of the second nanofiltration trapped concentrate and the monovalent salts in the second nanofiltration trapped concentrate are diafiltrated to the second nanofiltration diafiltrate. The electrical conductivity of the second nanofiltration trapped concentrate is reduced to less than 10µs; after mixing the second nanofiltration filtrate and the second nanofiltration diafiltrate, the electrical conductivity is less than 1000µs.

Step 6, performing reverse osmosis membrane process after mixing the second nanofiltration filtrate and the second nanofiltration diafiltrate to ensure that the electrical conductivity of the reverse osmosis filtrate is less than 1µs. The reverse osmosis filtrate can be used as diafiltration water for the first diafiltration process and the second diafiltration process.

Table 2 below shows the NMMO content in each of the above stages.

**Table 2. NMMO content in each of the stages**

| Stage Name | NMMO Content (x 10g/l) | Volume of Diafiltrate |
|---|---|---|
| Ultrafiltration trapped concentrate | 15.78 | - |
| Ultrafiltration filtrate | 15.78 | - |
| First nanofiltration trapped concentrate | 14.35 | - |
| First nanofiltration filtrate | 12.7 | - |
| First nanofiltration diafiltrate obtained after 1-time diafiltration | 9.5 | 1 time the volume of the first nanofiltration trapped concentrate |
| First nanofiltration trapped concentrate obtained after 1-time diafiltration | 11.4 | |
| First nanofiltration diafiltrate obtained after 2-time diafiltration | 2.56 | 2 times the volume of the first nanofiltration trapped concentrate |
| First nanofiltration trapped concentrate obtained after 2-time diafiltration | 5.48 | |
| First nanofiltration diafiltrate obtained after 3-time diafiltration | 1.0 | 4 times the volume of the first nanofiltration trapped concentrate |
| First nanofiltration trapped concentrate obtained after 3-time diafiltration | 1.0 | |
| First nanofiltration diafiltrate obtained after 4-time diafiltration | 0.8 | 10 times the volume of the first nanofiltration trapped concentrate |
| First nanofiltration trapped concentrate obtained after 4-time diafiltration | 0.8 | |

The above second nanofiltration trapped concentrate (without the second nanofiltration diafiltration process) is prepared to a solution with NMMO mass content of 19.8%. Properties of the solution are analyzed and detected as follows:
Mass content of the NMMO: 19.8%
Total carbohydrate content: 5.5 ppm
Electrically conductivity: 76µs/cm
Content of N-methylmorpholine: 130 ppm
Content of Morpholine: 34 ppm
Content of iron ions: 0.001 ppm
Content of copper ions: 0.001 ppm
Turbidity: 0 NTU
Chromaticity: 180

HPLC analysis shows that multiple impurity peaks with different content still present in the lyocell fiber coagulation bath after combined membrane separation process.

In all the embodiments of the invention below, the second nanofiltration trapped concentrate treated by combined membrane separation process in this embodiment is used as raw materials for following crystallization and purification steps.

### Embodiment 3

This embodiment mainly analyzes the impact of crystallization yields of NMMO on the purity of NMMO crystal.

Evaporating the lyocell coagulation bath in the embodiment 2, which is performed with combined carbohydrate removal membrane separation process, by vacuum heating until the mass concentration of NMMO of 63.5%, 65.2%, 66.5%, 67.9% and 69.2% respectively and then cooling crystallization is performed respectively. The concentrated solutions of the coagulation baths at each concentration start to cool down at 37°C. When temperatures of the solutions with NMMO concentrations of 63.5%, 65.2%, 66.5%, 67.9%, and 69.2% are cooled down to 31.2 °C, 32.1 °C, 33.2 °C, 34.2 °C, and 35.1 °C respectively, seed crystals pre-soaked in NMMO saturated solution are added and the amount of the seed crystals added is 0.15% of the mass of the NMMO in the concentrated solutions of the coagulation baths. After the seed crystals are added, the cooling rate is controlled at 1-2°C/ hour until the temperature drops to 30°C, and then the cooling rate is accelerated to 3-4°C/ hour. When the temperature drops to 25°C, stop the crystallization. Separating the mother solution from the crystals by rapid vacuum filtration and the crystal mother solution is collected. The viscosity of the mother solution is low. Comparing with the crystal mother solution of the lyocell coagulation bath in embodiment 1, which is not performed with combined carbohydrate removal membrane separation process, the viscosity is significantly reduced. It is easy to separate the crystal mother solution from the crystals by vacuum filtration. The crystals are rinsed with a significant amount of high-purity NMMO aqueous solution with a concentration of 59%, the crystals are collected to be weighed and calculated for crystallization yield, and then the crystal purity (residual rate of impurity) is analyzed and detected.

The obtained crystals are added with water separately to prepare an aqueous solution with NMMO mass concentration of 19.8% and then undergo multiple analyses and detections. Table 3 shows the impact of crystallization yields on purity of NMMO crystal.

**Table 3. The impact of crystallization yields on purity of NMMO crystal**

| Initial concentration of crystallization solution | Crystallization yield | Residual rate of impurity (detection at UV wavelength of 200nm) | Residual rate of impurity (detection at UV wavelength of 220nm) | Electrical conductivity (µs/cm) |
|---|---|---|---|---|
| Coagulation bath with combined membrane separation process | | 100% | 100% | 76 |
| 63.5% | 27% | 0.06% | 0.02% | 11.3 |
| 65.2% | 43% | 0.06% | 0.02% | 11.5 |
| 66.5% | 52% | 0.06% | 0.02% | 11.7 |
| 67.9% | 65% | 0.07% | 0.03% | 12.7 |
| 69.2% | 75% | 0.09% | 0.04% | 14.9 |

As shown in table 3, the crystallization can obtain NMMO crystal with very high purity and separate them from various impurities in the lyocell fiber coagulation bath. The electrical conductivity is also greatly reduced. When the crystallization yield is lower than 65%, the residual rate of the impurity in the NMMO crystal generally remains the same with the increase of the crystallization yield. When the crystallization yield is greater than 52%, the electrical conductivity of the crystal slightly increases with the increase of the crystallization yield.

### Embodiment 4

This embodiment mainly analyzes the impact of cooling rates on the purity of NMMO crystal.

Evaporating the lyocell coagulation bath in the embodiment 2, which is performed with combined membrane separation preprocess, by vacuum heating until the mass concentration of NMMO of 69.2%. When the temperature of the concentrated solution of the coagulation bath drops to 34.2 °C, NMMO seed crystals pre-soaked in NMMO saturated solution are added and the amount of the seed crystals added is 0.15% of the mass of the NMMO in the concentrated solution. The concentrated solution is divided into four portions and crystallized at the following different cooling rates separately after the seed crystals are added (34.2 °C). When the crystallization solutions are cooled down to 25 °C, performing vacuum filtration and processing the crystals according to the method in Embodiment 3.

### Cooling rates:

Cooling rate 1: cooling rate is controlled at 1 °C/hour from 34.2 °C to 30 °C, and cooling rate is controlled at 4 °C/hour between 30-25 °C.
Cooling rate 2: cooling rate is controlled at 2 °C/hour from 34.2 °C to 30 °C, and cooling rate is controlled at 4 °C/hour between 30-25 °C.
Cooling rate 3: cooling rate is controlled at 2 °C/hour from 34.2 °C to 30 °C, and cooling rate is controlled at 2 °C/hour between 30-25 °C.
Cooling rate 4: cooling rate is controlled at 4 °C/hour from 34.2 °C to 30 °C, and cooling rate is controlled at 4 °C/hour between 30-25 °C.

The obtained NMMO crystal is added with water to prepare an NMMO aqueous solution with mass concentration of 19.8% and then undergo multiple analyses and detections. Table 4 shows the impact of cooling rates on the purity of NMMO crystal.

**Table 4. The impact of cooling rates on the purity of NMMO crystal**

| Cooling rate | Crystallization yield | Residual rate of impurity (detection at UV wavelength of 200nm) | Residual rate of impurity (detection at UV wavelength of 220nm) | Electrical conductivity (µs/cm) |
|---|---|---|---|---|
| Cooling rate 1 | 75% | 0.07% | 0.04% | 14.8 |
| Cooling rate 2 | 75% | 0.07% | 0.04% | 14.9 |
| Cooling rate 3 | 75% | 0.07% | 0.04% | 14.9 |
| Cooling rate 4 | 76% | 0.09% | 0.06% | 15.1 |

As shown in table 4, during the initial crystallization stage (from the temperature of initial appearance of crystals to 30 °C), the cooling rate should not be too fast, and it is best to control the cooling rate at 1-2°C/hour, otherwise the crystal purity will slightly decrease and the electrical conductivity will slightly increase; during late stage of cooling crystallization, the cooling rate can be appropriately accelerated. The main reason is that the solubility of NMMO crystal varies greatly with temperature in the high-temperature region. Therefore, too rapid cooling leads to a high degree of supersaturation of the solution, resulting in that more mother solution may be embedded in the crystal and the crystal purity may be reduced; during late stage of crystallization, the crystallization temperature is relatively low, and the solubility trend of NMMO slows down with temperature, so the cooling rate can be appropriately accelerated.

### Embodiment 5

This embodiment mainly studies the impact of whether seed crystals are added, and the amount of seed crystals added on particle size of NMMO crystal and crystal purity.

Evaporating the lyocell coagulation bath in the embodiment 2, which is performed with combined membrane separation preprocess, by vacuum heating until the NMMO concentration of 69.4%. The concentrated solution is divided into 6 portions. When the temperatures of the concentrated solutions drop to 34.2 °C, NMMO seed crystals pre-soaked in NMMO saturated solution are added respectively. The amount of the seed crystals added as a percentage of the NMMO mass in the concentrated solution is: 0 (no seed crystals added), 0.05%, 0.1%, 0.2%, 0.3%, and 0.6%, and then crystallization and processing of crystals are performed according to the method in Embodiment 3.

The obtained crystalline NMMO is added with water to prepare an aqueous solution with NMMO mass concentration of 19.8% and then undergo multiple analyses and detections.

Table 5 shows impact of the amount of seed crystals added on purity of NMMO crystal and crystal particle size.

**Table 5. Impact of the amount of seed crystals added on purity and particle size of NMMO crystal**

| Amount of seed crystals added | Crystallization yield | Residual rate of impurity (detection at UV wavelength of 200nm) | Residual rate of impurity (detection at UV wavelength of 220nm) | Electrical conductivity (µs/cm) | Crystal particle size, distribution of particle size |
|---|---|---|---|---|---|
| 0 | 78% | 0.13% | 0.08% | 15.9 | Uneven distribution of particle size |
| 0.05% | 77% | 0.08% | 0.04% | 15.0 | Uneven distribution of particle size |
| 0.1% | 77% | 0.07% | 0.04% | 14.8 | Large particles with uniform distribution |
| 0.2% | 77% | 0.07% | 0.04% | 14.8 | Large particles with uniform distribution |
| 0.3% | 77% | 0.07% | 0.04% | 14.8 | Relatively large particles with uniform distribution |
| 0.6% | 77% | 0.08% | 0.05% | 15.0 | Relatively small particles with uniform distribution |

As shown in table 5, the amount of seed crystals added has major impact on crystallization particle size. When no seed crystals are added, or the amount of seed crystals added is relatively small (0.05%), the distribution of crystal particle size is uneven, indicating that adding insufficient amount of seed crystals leads to a relatively high degree of supersaturation of solution system and small crystal nucleus is spontaneously formed, resulting in a relatively low crystal purity. If enough amount of seed crystals is added, the distribution of crystal particles becomes uniform, and the crystal purity is high. The larger the amount of seed crystals added, the smaller the particles; however, if excessive amount of seed crystals (above 0.6%) is added, the resulting crystal particles will be too small, and the surface of the particles may be contaminated with more impurities from the mother solution, resulting in a slightly decrease in the crystal purity. At the same time, the small particle size of the crystals may affect the filtration speed of the crystals. The optimal amount of seed crystals added is 0.1-0.3% of the NMMO mass in the concentrated solution.

### Embodiment 6

This embodiment mainly studies the impact of secondary crystallization (recrystallization) on the purity of NMMO crystal.

According to the method in embodiment 3, concentrating the lyocell coagulation bath in the embodiment 2, which is performed with combined membrane separation preprocess until the NMMO mass concentration of 69.2% and then performing crystallization and purification (first crystallization). Adding water to crystals obtained from the first crystallization to prepare a NMMO aqueous solution with concentration of 69.9% and performing recrystallization according to the process in the embodiment 3. Crystals from the first crystallization and recrystallization crystals are collected to be weighed and calculated for crystallization yield, and the crystal purity (residual rate of impurity) is analyzed and detected. Crystals from the first crystallization and recrystallization crystals are added with water respectively to prepare aqueous solutions with NMMO concentration of 19.8%, and then undergo multiple analyses and detections. Table 6 shows the impact of recrystallization on the purity of NMMO crystal.

**Table 6. Data of the impact of recrystallization on the purity of NMMO crystal**

| Crystallization order | Crystallization yield | Color | Residual rate of impurity (detection at UV wavelength of 200nm) | Residual rate of impurity (detection at UV wavelength of 220nm) | Electrical conductivity (µs/cm) |
|---|---|---|---|---|---|
| First crystallization | 75% | Colorless and transparent | 0.09% | 0.04% | 14.9 |
| Recrystallization | 81% | Colorless and transparent | 0.02% | 0.01% | 12.4 |

As shown in table 6, the recrystallization can further purify the NMMO, reduce the content of organic impurities in NMMO crystal, and reduce the electrical conductivity of the NMMO crystal to 12.4µs/cm.

### Embodiment 7

This embodiment mainly studies the effects of recovering NMMO from the residual mother solution of the first crystallization through second crystallization.

Evaporating the mother solution separated from the first crystallization of the concentrated solution of the lyocell coagulation bath in the embodiment 6 by heating under reduced pressure until the NMMO mass concentration of 70.1% and performing crystallization according to the crystallization process and condition in embodiment 3 (seed crystals are added when the temperature of the mother solution is 35.3°C). The obtained crystals are added with water to prepare an aqueous solution with NMMO mass concentration of 19.8% and then undergoes multiple analyses and detections. Table 7 shows the result of performing crystallization again to the mother solution of the first crystallization of the concentrated solution of the lyocell fiber coagulation bath.

**Table 7. The result of performing crystallization again to the mother solution of the first crystallization of the concentrated solution of the lyocell fiber coagulation bath**

| | Crystallization yield | Residual rate of impurity (detection at UV wavelength of 200nm) | Residual rate of impurity (detection at UV wavelength of 220nm) | Electrical conductivity (µs/cm) |
|---|---|---|---|---|
| Mother solution of the first crystallization is concentrated to 70.1% of NMMO concentration | 84% | 0.13% | 0.08% | 84.1 |

As shown in table 7, the mother solution of the first crystallization is concentrated again for crystallization. When the crystallization yield reaches 84%, the purity of the obtained crystals is still very high. The first and the second crystallizations can totally recover 96% of NMMO in the lyocell fiber coagulation bath in the embodiment; furthermore, the viscosity of the mother solution of the second crystallization is still not high. If the higher recovery rate of NNMO crystallization is needed, further concentration and crystallization of the mother solution of the second crystallization can be carried out, or the crystallization yield of the first and the second crystallization can be further improved.

### Industrial applications

In summary, the invention provides a purification method of NMMO in the lyocell fiber coagulation bath. The method adopts crystallization purification for the purification and the recovery, and the crystallization can be run at a temperature close to room temperature (25 ~ 35 °C). Results of HPLC analyses and detection show that the purity of the NMMO, which is obtained by the crystallization process and method provided in the invention, is very high. Multiple organic impurities including PG, PG oxides, carbohydrates, etc. present in the lyocell coagulation bath are almost completely removed. The purity of the NMMO recovered and purified by the invention is significantly higher than the purity of the NMMO purified and recovered by the method of ion-exchange resins, which is currently widely used. The purity of the NMMO in the invention is even close to the purity of commercially available NMMO products. Various impurities that cannot be removed by ion-exchange resins can be removed by the invention, which solves the problem that the current purification process of coagulation bath by using ion-exchange resins cannot remove many impurities in the lyocell spinning system and a long-term accumulation of the impurities affects the quality of the lyocell spinning. In addition, combining the membrane separation process with the crystallization process can further improve the recovery rate of NMMO and make the purification process easier to carry out and fit for industrial productions.

Using the crystallization and purification process provided in the invention, the resulting final crystal mother solution, which contains a large amount of impurities, can be disposed of by incineration. Since the recovery and purification method of the invention does not need to use ion-exchange resins, it completely solved the environmental problems in the current purification process using ion-exchange resin, such as the wastewater with high concentration of salt and COD content and spent ion-exchange resins. Moreover, the crystallization and purification process provided in the invention can remove almost all the impurities in the lyocell coagulation bath. The accumulation of impurities in the spinning system due to non-removable impurities will not happen. Hence, it is possible for lyocell fiber production to use broad-leaved forest pulps with high content of easily decomposed hemicellulose and even paper pulp with higher content of hemicellulose, which expands the source of raw material for lyocell pulp and alleviates or even solves fibrillation problem currently existing in lyocell fibers. The invention provides a green, low production cost and efficient recovery and purification method of NMMO in the lyocell fiber coagulation bath.

## Claims

1. A purification method of N-methylmorpholine N-oxide, which is used to purify N-methylmorpholine N-oxide in a lyocell fiber coagulation bath, wherein comprising steps of: performing cooling crystallization to the lyocell fiber coagulation bath between -20 °C and 78 °C to obtain NMMO hydrate crystals.

2. The purification method of N-methylmorpholine N-oxide according to claim 1, wherein comprising another step before performing the cooling crystallization to the lyocell fiber coagulation bath: removing carbohydrates in the lyocell fiber coagulation bath.

3. The purification method of N-methylmorpholine N-oxide according to claim 1, wherein comprising another step before performing the cooling crystallization to the lyocell fiber coagulation bath: performing at least one of microfiltration, ultrafiltration and nanofiltration to the lyocell fiber coagulation bath.

4. The purification method of N-methylmorpholine N-oxide according to claim 1, wherein comprising another step before performing the cooling crystallization to the lyocell fiber coagulation bath: mixing the lyocell fiber coagulation bath with coagulant aids to obtain a mixture to perform flocculation and sedimentation; then performing at least one of microfiltration, ultrafiltration and nanofiltration to the mixture.

5. The purification method of N-methylmorpholine N-oxide according to any one of claims 1-4, wherein concentrating the lyocell fiber coagulation bath before the cooling crystallization is performed until the mass concentration of N-methylmorpholine N-oxide in the lyocell fiber coagulation bath is 56.5% ~ 84.5%, and then the cooling crystallization is performed.

6. The purification method of N-methylmorpholine N-oxide according to claim 5, wherein the mass concentration of N-methylmorpholine N-oxide in the lyocell fiber coagulation bath is 56.5% ~ 72.2% by the concentration and the temperature of the cooling crystallization is between 25 °C ~ 40 °C.

7. The purification method of N-methylmorpholine N-oxide according to claim 1, wherein during the cooling crystallization process, a cooling rate is 1 ~ 2°C/ hour from an initial appearance of crystals to 30°C; the cooling rate is 3 ~ 4°C/ hour between -20°C ~ 30°C.

8. The purification method of N-methylmorpholine N-oxide according to claim 1, wherein seed crystals are added during the cooling crystallization process, and the amount of the seed crystals added is above 0.1% of the weight of the N-methylmorpholine N-oxide in the lyocell fiber coagulation bath.

9. The purification method of N-methylmorpholine N-oxide according to claim 1, wherein the cooling crystallization includes at least a primary cooling crystallization and a secondary cooling crystallization, the NMMO hydrate crystals and a crystal mother solution are obtained by the primary cooling crystallization, and the crystal mother solution is first concentrated and then subjected to the secondary cooling crystallization.

10. The purification method of N-methylmorpholine N-oxide according to claim 4, wherein performing the microfiltration to the mixture to obtain microfiltration filtrate; performing the ultrafiltration to the microfiltration filtrate to obtain an ultrafiltration trapped concentrate and an ultrafiltration filtrate; performing the cooling crystallization to the ultrafiltration filtrate; the filter aperture of the microfiltration is 0.5 ~ 5 µm; the ultrafiltration has molecular weight cut off 1000 ~ 100000.

11. The purification method of N-methylmorpholine N-oxide according to claim 10, wherein performing a first nanofiltration to the ultrafiltration filtrate first to obtain a first nanofiltration trapped concentrate and a first nanofiltration filtrate, and then performing the cooling crystallization to the first nanofiltration filtrate; the first nanofiltration has molecular weight cut off 300 ~ 1000.

12. The purification method of N-methylmorpholine N-oxide according to claim 11, wherein performing a second nanofiltration to the first nanofiltration filtrate first to obtain a second nanofiltration trapped concentrate and a second nanofiltration filtrate, and the N-methylmorpholine N-oxide is kept in the second nanofiltration trapped concentrate, then performing the cooling crystallization to the second nanofiltration trapped concentrate; the second nanofiltration has molecular weight cut off 100 ~ 200.

13. A N-methylmorpholine N-oxide hydrate crystal obtained by the purification method according to any one of claims 1-12.

14. A purification system of N-methylmorpholine N-oxide, which is used to purify N-methylmorpholine N-oxide in a lyocell fiber coagulation bath, wherein comprising:
a crystallization device configured to crystallization process of the lyocell fiber coagulation bath;
a control device connected to the crystallization device to control crystallization conditions in the crystallization device.

15. The purification system of N-methylmorpholine N-oxide according to claim 14, wherein further comprises:
at least one of a microfiltration device, an ultrafiltration device and a nanofiltration device connected to the crystallization device and the control device, and the lyocell fiber coagulation bath flows into at least one of the microfiltration device, the ultrafiltration device and the nanofiltration device for processing to obtain a filtrate and the filtrate flows into the crystallization device for crystallization.

16. The purification system of N-methylmorpholine N-oxide according to claim 15, wherein further comprises:
a flocculation and sedimentation device, and the lyocell fiber coagulation bath and coagulant aids flow into the flocculation and sedimentation device to obtain a mixture to perform flocculation and sedimentation;
the microfiltration device is connected to the flocculation and sedimentation device so that the mixture flows into the microfiltration device to filter solids and obtain microfiltration filtrate;
the ultrafiltration device is connected to the microfiltration device and the microfiltration filtrate flows into the ultrafiltration device to obtain an ultrafiltration trapped concentrate and an ultrafiltration filtrate;
the nanofiltration device is connected to the ultrafiltration device and the crystallization device respectively, the ultrafiltration filtrate flows into the nanofiltration device to obtain a nanofiltration trapped concentrate and a nanofiltration filtrate, and the N-methylmorpholine N-oxide is kept in the nanofiltration trapped concentrate, which flows into the crystallization device for crystallization.

17. The purification system of N-methylmorpholine N-oxide according to claim 16, wherein the nanofiltration device comprises a first nanofiltration device and a second nanofiltration device;
the first nanofiltration device is connected to the ultrafiltration device, and the ultrafiltration filtrate flows into the first nanofiltration device to obtain a first nanofiltration trapped concentrate and a first nanofiltration filtrate;
the second nanofiltration device is connected to the first nanofiltration device and the crystallization device respectively, the first nanofiltration filtrate flows into the second nanofiltration device to obtain a second nanofiltration trapped concentrate and a second nanofiltration filtrate, and the N-methylmorpholine N-oxide is kept in the second nanofiltration trapped concentrate, which flows into the crystallization device for crystallization.
